# DEMANDE DE BREVET EUROPEEN

(11) **EP 2 862 923 A1**
(43) Date de publication de la demande: **22.04.2015**
(21) Numéro de dépôt: 14187759.7
(22) Date de dépôt: 06.10.2014
(51) Int. Cl.: C12M 1/107, C12M 1/00, C12M 1/34

(54) **Procédé de production de biométhane pour injection dans un réseau de gaz à partir d'une pluralité de sites de production et ensemble de dispositifs pour sa mise en oeuvre**

(30) Priorité: 15.10.2013 FR 1360013
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: Paget, Nicolas, 38000 GRENOBLE (FR); Roux, Pierre, 38960 SAINT ETIENNE DE CROSSEY (FR)
(74) Mandataire: Beroud, Amandine

(57) **Abrégé**

La présente invention concerne un procédé de production de biométhane destiné à alimenter un réseau de gaz naturel à partir de n installations Iᵢ de production de biogaz, avec i variant de 1 à n, dans lequel chacune des installations produit et stocke du biogaz qui est collecté sur chacune des installations via un dispositif mobile de collecte, le biogaz collecté est épuré de sorte à produire du biométhane qui est par la suite injecté dans un réseau de gaz naturel.

Le procédé selon l'invention prévoit en outre de stocker du gaz pour assurer la continuité de la fourniture du biométhane au réseau durant la tournée de collecte.

## Description

La présente invention concerne un procédé de production de biométhane destiné à alimenter un réseau de gaz naturel à partir d'une pluralité d'installations de production de biogaz, comprenant les étapes de production, stockage, épuration en biométhane et alimentation du réseau, ainsi qu'un ensemble de dispositifs pour la mise en oeuvre du procédé.

Dans le cadre de sa valorisation, le biométhane - en tant que substitut renouvelable au gaz naturel ayant les mêmes caractéristiques que celui-ci - peut être injecté dans un réseau de distribution ou de transport de gaz naturel qui permet de relier producteurs de gaz et consommateurs.

Un réseau de distribution ou de transport de gaz naturel permet d'approvisionner des consommateurs en gaz naturel. Le réseau est maintenu à une pression comprise entre 2 et 6 bars pour la distribution, 15 et 25 bars pour la distribution moyenne pression et 25 à 80 bars pour le transport.

Le biogaz est un gaz produit par la fermentation naturelle ou artificielle de matières organiques végétales ou animales (la méthanisation). Il contient majoritairement du méthane (CH₄) du dioxyde de carbone (CO₂), mais également - en moindre proportion - de l'eau, de l'azote, de l'hydrogène sulfuré, de l'oxygène, ainsi que des composés organiques autres, à l'état de traces.

Selon les matières organiques et les techniques utilisées, les proportions des composants diffèrent, mais en moyenne le biogaz comporte, sur gaz sec, de 30 à 75% de méthane, de 15 à 60% de CO₂, de 0 à 5% d'azote, de 0 à 5% d'oxygène et des composés traces.

Aux valorisations - essentiellement sur site ou à proximité - du biogaz, s'est ajoutée plus récemment celle du biogaz épuré aux spécifications du gaz naturel. Le biométhane peut être utilisé en tant que substitut non fossile du gaz naturel complétant ainsi les ressources de gaz naturel avec une partie renouvelable produite au coeur des territoires. Il est utilisable pour exactement les mêmes usages.

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Pour produire et utiliser le biométhane en tant que gaz naturel renouvelable dans les réseaux de gaz naturel, deux acteurs principaux interviennent : le premier de ces acteurs est le producteur de biométhane, le second acteur est le distributeur de gaz naturel qui gère le réseau de gaz.

Pour avoir la possibilité d'injecter du biométhane dans un réseau de gaz existant, il est nécessaire de prévoir l'installation d'un poste de comptage (poste d'injection GRdF, GRT Gaz par exemple) et d'assurer la connexion au réseau proche. La pression du gaz dans le réseau peut varier et imposera parfois une compression supplémentaire pour permettre l'injection.

Aussi, lorsqu'un producteur de biogaz souhaite utiliser son gaz pour produire du biométhane à injecter dans un réseau, il doit faire face dans tous les cas à des coûts d'installation et d'entretien du système d'injection; il arrive aussi dans certains cas que des contraintes supplémentaires soient imposées au producteur de biogaz :
- contraintes techniques de l'opérateur de réseau qui nécessitent des investissements conséquents, et/ ou
- volume minimum de biogaz à produire qui souvent dépasse la capacité de production des petits producteurs (agricoles, particuliers ou autres).

Il n'existe pas à ce jour pour ces petits producteurs de biogaz de solution qui leur permette de produire du biogaz dont la finalité est l'injection dans un réseau de gaz naturel.

Il y a donc un besoin d'une solution de production de biométhane - disponible pour être injecté dans un réseau - qui permette de traiter une pluralité de productions de biogaz de faibles volumes en vue de produire du biométhane pour un coût limité pour les producteurs.

Il y a aussi un besoin, pour les gestionnaires de réseau de gaz naturel, de disposer d'approvisionnements suffisants et fiables en biométhane en vue d'augmenter la part de gaz renouvelable dans le gaz circulant dans le réseau.

Il y a donc un besoin d'une solution qui assure aux producteurs la valorisation de leurs productions de biogaz trop faibles pour être valorisées individuellement et qui assure l'approvisionnement en biométhane d'un réseau de gaz naturel - de distribution ou de transport - à partir d'une pluralité de productions de biogaz.

Selon l'invention, il est pour cela proposé un procédé de production de biométhane destiné à alimenter un réseau de gaz naturel à partir de n installations Iᵢ de production de biogaz, avec i variant de 1 à n, comprenant au moins les étapes suivantes :
- pour chacune des installations Iᵢ de production de biogaz une étape **aᵢ** de production de biogaz Pᵢ,
- pour chacune des installations Iᵢ de production de biogaz une étape **bᵢ** de stockage du biogaz produit Pᵢ,
- une étape **c** de collecte de l'ensemble des biogaz Pᵢ produits et stockés sur chacune des installations Iᵢ via un dispositif mobile de collecte,
- une étape **d** d'épuration du biogaz collecté de sorte à produire du biométhane,
- une étape **e** d'injection du biométhane dans le réseau de gaz naturel.

Le procédé ci-dessus fonctionne de la manière suivante : chacune des installations Iᵢ de production de biogaz produit son biogaz Pᵢ et le stocke entre deux collectes successives; le dispositif mobile de collecte circule entre les installations Iᵢ de production. Lorsque le dispositif de collecte arrive sur le site de l'installation Iᵢ, il collecte le gaz présent dans le stockage de l'installation puis rejoint l'installation Iᵢ₊₁ pour y collecter de même le biogaz produit et préalablement stocké lui aussi. Le biogaz collecté (somme des biogaz Pᵢ) est traité pour produire du biométhane. La transformation n'est pas assurée individuellement par chaque petit producteur, un dispositif d'épuration associé à la collecte traite l'ensemble des productions de biogaz ce qui réduit considérablement le coût pour le producteur de biogaz. L'approvisionnement du réseau se fait alors à partir du biométhane issu de l'ensemble des productions, il est plus fiable, tant en terme de qualité que de volume.

Selon les cas le procédé de l'invention peut comprendre tout ou partie des caractéristiques ci-après.
- Pour au moins une partie des installations Iᵢ de production de biogaz, le biogaz Pᵢ subit une étape de compression avant d'être stocké sur le site de l'installation ; le stockage du biogaz chez le producteur peut en effet être un stockage à la pression du méthaniseur de l'installation de production, mais le producteur peut privilégier un stockage à une pression supérieure afin de limiter le volume du stockage avant collecte. La capacité totale de stockage de biogaz chez le producteur est adaptée à la fréquence de passage du dispositif de collecte ; le stockage permettra en général deux jours de stockage, cependant, sa capacité pourra être limitée si la rotation de collecte est courte.
- En fonction des besoins, l'injection de biométhane dans le réseau est continue ou discontinue. Afin que l'injection dans le réseau puisse se faire en continu, il est nécessaire de prévoir de stocker du gaz sous pression après collecte; à cet effet, un stockage tampon haute pression de gaz après collecte est prévu, ou plusieurs.

En particulier, il pourra s'agir d'un stockage du biogaz collecté, et/ou d'un stockage de biogaz partiellement purifié, et/ou d'un stockage de biométhane. Du gaz est préférentiellement stocké à proximité du poste d'injection. Grâce à ce stockage tampon, il n'y aura pas de rupture de l'approvisionnement du réseau durant la collecte. Ainsi, selon les cas, le procédé de l'invention peut comprendre, soit une étape de compression du biogaz suivie d'une étape de stockage du biogaz sous pression préalablement à l'étape de purification, ou une étape de compression de biogaz partiellement purifié suivie d'une étape de stockage du biogaz partiellement purifié sous pression lorsqu'une première partie de la purification est réalisée via un dispositif de purification mobile, ou encore une étape de compression du biométhane suivie d'une étape de stockage du biométhane sous pression lorsque l'étape de purification est réalisée intégralement via un dispositif de purification mobile.

Selon un second aspect de l'invention, celle-ci concerne un ensemble de dispositifs aptes à mettre en oeuvre le procédé de l'invention.

Elle concerne ainsi un ensemble de dispositifs destiné à alimenter un réseau de gaz naturel à partir de n installations Iᵢ de production de biogaz, avec i variant de 1 à n, comprenant au moins :
- n installations Iᵢ aptes à assurer n productions Pi de biogaz,
- pour chaque installation Iᵢ, un moyen de stockage du biogaz produit,
- pour au moins une partie des installations Iᵢ, un moyen de compression du biogaz Pᵢ préalablement à son stockage,
- un dispositif mobile de collecte apte à assurer la collecte de l'ensemble des biogaz stockés Pi,
- un dispositif d'épuration du biogaz collecté de sorte à produire du biométhane,
- un dispositif d'injection du biométhane dans le réseau de gaz naturel à alimenter,

Le dispositif mobile de collecte de biogaz chez les producteurs contient de préférence un moyen de compression pour le remplissage d'un moyen de stockage de gaz mobile intégré. Ce moyen de stockage mobile est de préférence un camion citerne ou équipé de cadres de bouteilles adaptées au transport de gaz sous pression, mais d'autres moyens de transport adaptés à des situations particulières peuvent être envisagés (transport fluvial, ferroviaire ou autre). Les transports devront naturellement respecter les règlementations en vigueur ; pour le transport de gaz par camion par exemple, il faudra ainsi se référer à la réglementation routière en vigueur.

Le dispositif d'épuration du biogaz pour produire le biométhane pourra comporter tout ou partie des modules ci-après utilisant des technologies d'épuration classiques ; on cite ici à titre d'exemples certains moyens et technologies pouvant être utilisés :
- un module de séchage utilisant une technologie parmi les suivantes : tamis moléculaire, échangeur refroidisseur, membranes;
- un ou des module(s) d'abattement des polluants tels que H₂S, COv, COvSi, NH₃ , utilisant charbons actifs, lavage à la soude régénératif ou non, membrane, lavage à l'eau, oxyde de fer, tamis régénérable ou non, lavage biologique, lavage aux amines, lavage au CO₂ liquide, etc ;
- un module de séparation CH₄ / CO₂ utilisant membranes sélectives, lavage à l'eau, adsorption à pression modulée (PSA), lavage aux amines, lavage au CO₂ liquide, système cryogénique, etc ;
- des modules de traitement des effluents et des évents, utilisant pour le traitement de ces derniers oxydateur thermique, chaudière gaz, filtre biologique, etc ;
- un module de compression optionnel pour stockage.

Selon un mode de réalisation particulier de l'invention, tout ou partie du dispositif d'épuration du biogaz collecté peut être mobile. Les différents moyens de traitement ou une partie d'entre eux peuvent être mobiles et disposés sur un moyen de transport commun entre eux et/ou avec des moyens de stockage de gaz ou être mobiles et séparés.

Alternativement, l'ensemble du dispositif d'épuration du biogaz collecté étant fixe, il est préférentiellement installé à proximité du poste d'injection du biométhane dans le réseau.

L'installation peut comprendre un ou plusieurs moyens de stockage de gaz après collecte.

En particulier, lorsque tout ou partie du dispositif d'épuration est mobile, le gaz stocké pourra être du biogaz partiellement purifié, mais sera en général du biométhane sous pression, le moyen de stockage étant installé à proximité du poste d'injection. Alternativement, quand le dispositif d'épuration est fixe, le stockage pourra être un stockage de biogaz.

Le dispositif d'injection du biométhane vers le réseau de gaz doit comporter des moyens d'analyse et de comptage, nécessaires pour valider la qualité et la facturation ; il se situe au niveau d'un poste d'injection défini par l'opérateur du réseau (garant de la qualité et du comptage du biométhane transféré au réseau).

L'invention va maintenant être mieux comprise grâce à la description suivante faite en références à la figure jointe.

La figure présente un schéma de principe illustrant différents éléments de l'invention à partir de n sites de production référencés Ii.

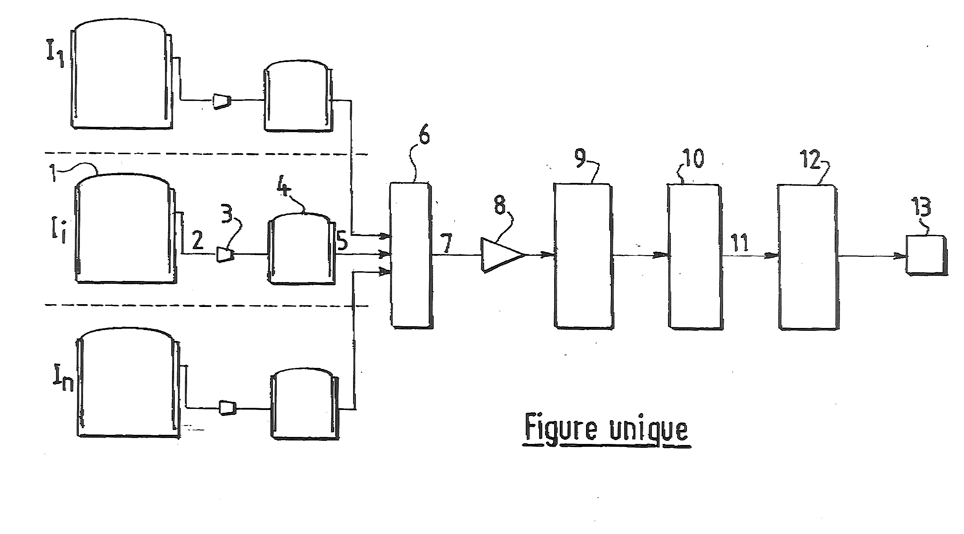

Dans un but de simplicité, la figure 1 ne reproduit, parmi les n sites de production que trois sites identifiés I₁, Ii et In. Toujours dans un but de simplicité, ne sont référencés que les éléments essentiels présents de l'installation Iᵢ. Le fonctionnement de l'invention est décrit en se référant à ce site Iᵢ

Selon le schéma de la figure 1, chaque site de production Iᵢ comprend un méthaniseur **1,** produisant un biogaz **2,** le biogaz est comprimé dans un compresseur **3,** puis stocké dans un réservoir **4** de stockage du biogaz Pᵢ produit sur le site, ces différents éléments étant reliés entre eux par des canalisations transportant le biogaz produit. Le biogaz Pᵢ est comprimé pour occuper un volume de stockage plus faible; cependant, la compression du biogaz n'est pas obligatoire - on peut aussi concevoir que certains sites de production ne compriment pas leur biogaz, tandis que d'autres le compriment. Le biogaz Pᵢ produit par chaque site Ii référencé **5** est collecté via le moyen de collecte **6.** Selon la variante représentée sur la figure, le biogaz collecté 7 est comprimé via le compresseur **8** et stocké dans un réservoir de stockage **9** avant d'être épuré via le dispositif d'épuration **10.** Le dispositif d'épuration **10** est situé à proximité du lieu d'injection dans le réseau. Le dispositif d'épuration est unique pour l'ensemble des installations de production. Le biométhane produit **11** alimente le système **12** d'injection et de comptage du biométhane situé au niveau du poste d'injection pour être contrôlé, compté et injecté dans le réseau..

Le schéma ne reproduit pas l'ensemble des éléments utiles à la production et au traitement du biogaz et du biométhane, mais essentiellement les éléments utiles à la compréhension de l'invention; on s'est ainsi abstenu d'indiquer les éléments contenus dans le dispositif d'épuration, ainsi que dans le poste d'injection, le détail de ces éléments n'étant pas nécessaire pour décrire le fonctionnement de l'invention et ces éléments sont connus de l'homme du métier.

## Revendications

1. Procédé de production de biométhane destiné à alimenter un réseau de gaz naturel à partir de n installations Ii de production de biogaz, avec i variant de 1 à n, comprenant au moins les étapes suivantes :
• pour chacune des installations Ii de production de biogaz une étape **aᵢ** de production de biogaz Pi,
• pour chacune des installations Ii de production de biogaz une étape **bᵢ** de stockage du biogaz produit Pi,
• une étape **c** de collecte de l'ensemble des biogaz Pi produits et stockés sur chacune des installations Ii via un dispositif mobile de collecte circulant entre les installations Ii,
• une étape **d** d'épuration du biogaz collecté de sorte à produire du biométhane,
• une étape **e** d'injection du biométhane dans le réseau de gaz naturel.

2. Procédé selon la revendication 1 dans lequel pour au moins une partie des installations Ii de production de biogaz, le biogaz Pi subit une étape de compression avant d'être stocké sur le site de l'installation.

3. Procédé selon la revendication 1 ou 2 comprenant une étape de compression du biogaz collecté suivie d'une étape de stockage du biogaz sous pression préalablement à l'étape de purification, ou d'une étape de compression de biogaz partiellement purifié suivie d'une étape de stockage du biogaz partiellement purifié sous pression lorsqu'une première partie de la purification est réalisée via un dispositif de purification mobile, ou d'une étape de compression du biométhane suivie d'une étape de stockage du biométhane sous pression lorsque l'étape de purification est réalisée intégralement via un dispositif de purification mobile.

4. Ensemble de dispositifs pour mettre en oeuvre le procédé de la revendication 1 ou 2 destiné à alimenter un réseau de gaz naturel à partir de n installations Ii de production de biogaz, avec i variant de 1 à n, comprenant au moins :
• n installations Ii aptes à assurer n productions Pi de biogaz,
• pour chaque installation Ii, un moyen de stockage du biogaz produit,
• pour au moins une partie des installations Ii, un moyen de compression du biogaz Pi préalablement à son stockage,
• un dispositif mobile de collecte apte à assurer la collecte de l'ensemble des biogaz stockés Pi en circulant entre les installations Ii,
• un dispositif d'épuration du biogaz collecté de sorte à produire du biométhane,
• un dispositif d'injection du biométhane dans le réseau de gaz naturel à alimenter.

5. Ensemble de dispositifs selon la revendication 4 dans lequel tout ou partie du dispositif d'épuration du biogaz collecté est mobile.

6. Ensemble de dispositifs selon la revendication 4 dans lequel le dispositif d'épuration du biogaz collecté est fixe, et de préférence placé à proximité du poste d'injection du biométhane dans le réseau.

7. Ensemble de dispositifs selon l'une des revendications 4 à 6 comprenant soit un moyen de compression du biogaz et un moyen de stockage du biogaz sous pression en amont du dispositif fixe d'épuration, ou alternativement un moyen de compression et un moyen de stockage du biogaz partiellement purifié sous pression situé intermédiairement entre un dispositif de purification partiel mobile et un dispositif complémentaire de purification fixe ou un moyen de compression du biométhane et un moyen de stockage du biométhane sous pression en aval du dispositif de purification lorsque celui-ci est mobile.
